# EUROPÄISCHE PATENTSCHRIFT

(11) **EP 2 882 498 B1**
(45) Veröffentlichungstag und Bekanntmachung des Hinweises auf die Patenterteilung: **30.08.2017**
(21) Anmeldenummer: 13739636.2
(22) Anmeldetag: 10.07.2013
(51) Int. Cl.: A61Q 5/06, A61K 8/73, A61K 8/81, A61K 8/92

(54) **MITTEL UND VERFAHREN ZUR TEMPORÄREN VERFORMUNG KERATINHALTIGER FASERN**
AGENT AND METHOD FOR THE TEMPORARY DEFORMATION OF KERATIN FIBRES
PRODUITS ET PROCÉDÉS POUR METTRE EN FORME TEMPORAIREMENT DES FIBRES KÉRATINIQUES

(30) Priorität: 13.08.2012 DE 102012214380
(43) Veröffentlichungstag der Anmeldung: 17.06.2015
(73) Patentinhaber: Henkel AG & Co. KGaA, 40589 Düsseldorf (DE)
(72) Erfinder: KNAPPE, Thorsten, 22869 Schenefeld (DE); NOLL, Marcus, 22850 Norderstedt (DE)
(86) Internationale Anmeldenummer: PCT/EP2013/064580
(87) Internationale Veröffentlichungsnummer: WO 2014/026804

(56) Entgegenhaltungen:
- EP-A1- 1 632 270
- DE-A1-102009 045 933
- DE-U1-202012 000 164
- DATABASE GNPD [Online] MINTEL; November 2010 (2010-11), "FishGlue Matt Styling Gel", XP002727218, Database accession no. 1504937

## Beschreibung

Die vorliegende Erfindung betrifft die Verwendung kosmetischer Mittel zur temporären Verformung keratinischer Fasern, insbesondere menschlicher Haare. Ein weiterer Gegenstand sind spezifische Mittel zur temporären Verformung keratinischer Fasern.

Stylingmittel zur Verformung keratinhaltiger Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle.

Die Mittel zur temporären Formgebung liegen in verschiedenen Konfektionsformen vor, wobei bei den Treibgas-freien Mitteln insbesondere Haarwachse und Haargele verbreitet sind. Diese Mittel unterscheiden sich aus Verbrauchersicht insbesondere auch hinsichtlich ihrer Produkthaptik. Die Haptik des Haarkosmetikums ist dabei in der Regel nicht das zufällige Ergebnis der Kombination spezifischer Wirkstoffe sondern ist neben weiteren sensorischen Eigenschaften wie der Optik und dem Geruch ein wesentliches Mittel zur Unterstützung Produkt- oder Markenversprechens und damit mitentscheidend für den kommerziellen Erfolg des Produktes.

Die Aufgabe der vorliegenden Patentanmeldung war es daher, kosmetische Mittel zur temporären Verformung keratinischer Fasern mit neuartiger Haptik und guten kosmetischen Eigenschaften zur Verfügung zur stellen. Die kosmetischen Mittels sollten sich in ihrer Haptik insbesondere von den bisher im Markt verbreiteten Gelen und Wachsen unterscheiden.

Es wurde festgestellt, dass sich diese Aufgaben durch die Kombination hydrophob modifizierter (Meth)acrylsäure Copolymer und hydrophob modifizierter Polysaccharide lösen lassen. Haarkosmetische Mittel auf Basis dieser Wirkstoffkombination zeichnen sich nicht nur durch eine einzigartige Haptik sondern auch durch gute festigende Eigenschaften, beispielsweise einen hohen Haltegrad bei geringer Klebrigkeit, hohe Flexibilität und gute Feuchtebeständigkeit - insbesondere Schweiß- und Wasserbeständigkeit - aus. Die Kombination hydrophob modifizierter (Meth)acrylsäure Copolymer und hydrophob modifizierter Polysaccharide eignet sich zudem für die Herstellung stabil viskoser Zusammensetzungen.

Kosmetische oder dermatologische Lichtschutzzubereitungen, die neben weiteren Inhaltsstoffen ein Acrylates/C10-30 Alkyl Acrylate Crosspolymer sowie ein Aluminium Starch Octenylsuccinat enthalten, werden in der deutschen Offenlegungssschrift DE 10 2010 063 842 A1 (Beiersdorf) offenbart.

Ein erster Gegenstand der vorliegenden Anmeldung ist daher die Verwendung gemäß Anspruch 1 einer kosmetischen Zusammensetzung, enthaltend
a) mindestens ein hydrophob modifiziertes (Meth)acrylsäure Copolymer;
b) mindestens ein hydrophob modifiziertes Polysaccharid.
zur temporären Verformung keratinhaltiger Fasern, insbesondere menschlicher Haare.

Die erfindungsgemäß verwendeten Mittel zeichnen sich durch eine gummiartige Konsistenz aus. Bedingt durch diese Konsistenz sind die verwendeten Mittel gut dosier- und applizierbar.

Die erfindungsgemäße Verwendung wird vorzugsweise dadurch realisiert, dass die keratinischen Fasern mit einer der zuvor beschriebenen kosmetischen Zusammensetzung beaufschlagt und temporär in ihrer Form fixiert werden. Die Beaufschlagung der keratinischen Fasern mit der kosmetischen Zusammensetzung erfolgt vorzugsweise durch Aufbringen der kosmetischen Zusammensetzung auf die Finger oder die Handinnenflächen und nachfolgendes Modellieren der keratinischen Fasern mit den Fingern oder Handinnenflächen, wodurch die kosmetische Zusammensetzung wenigstens anteilsweise von den Fingern oder Handinnenflächen auf die keratinischen Fasern übertragen werden.

Besonders bevorzugt wird die Verwendung einer kosmetischen Zusammensetzung, enthaltend
a) mindestens ein hydrophob modifiziertes (Meth)acrylsäure Copolymer;
b) mindestens ein hydrophob modifiziertes Polysaccharid.
zur temporären Verformung und Remodellierung keratinhaltiger Fasern, insbesondere menschlicher Haare.

Ein erster wesentlicher Bestandteil erfindungsgemäß verwendeter kosemtischer Zusammensetzungen ist das hydrophob modifizierte (Meth)acrylsäure Copolymer a). Der Gewichtsanteil des Copolymers a) am Gesamtgewicht der kosmetischen Zusammensetzung beträgt 0,05 bis 5,0 Gew.-%, besonders bevorzugt 0,1 bis 4,0 Gew.-% und insbesondere 0,2 bis 2,0 Gew.-%. Bevorzugte Copolymere a) haben eine verdickende Wirkung.

Als hydrophob modifizierte (Meth)acrylsäure Copolymere a) werden Copolymere verwendet, die aus
- mindestens einem Monomer (A1) aus der Gruppe Acrylsäure, Methacrylsäure, C₁-C₆-Alkylacrylsäureester, C₁-C₆-Alkylmethacrylsäureester,
- mindestens einem Monomer (A2) aus der Gruppe der der C₁₀₋₃₀Alkyl Acrylate, Steareth-20 Methacrylate, Beheneth-25 Methacrylate, Steareth-20 Itaconate, Ceteth-20 Itaconate, Palmeth-25 Acrylate oder C₁₀₋₃₀Alkyl PEG-20 Itaconate
gebildet werden.

Weitere bevorzugte hydrophob modifizierte (Meth)acrylsäure Copolymere a) werden neben den zuvor beschreibenen Monomeren (A1) und (A2) aus mindestens einem Monomer (A3) aus der Gruppe der ungesättigten Amingruppen-haltigen Monomere gebildet.

Als Monomer (A3) werden vorzugsweise Monomere aus der Gruppe Acrylamid, Methacrylamid, Mono-(C₁-C₄)-alkylamino(C₁-C₄)-alkylacrylat, Di-(C₁-C₄)-alkylamino(C₁-C₄)-alkylacrylat, Mono-(C₁-C₄)-alkylamino(C₁-C₄)-alkylmethacrylat, Di-(C₁-C₄)-alkylamino(C₁-C₄)-alkylmethacrylat eingesetzt. Beispielhafte und bevorzugte Monomer (A2) sind 2-(N,N-Dimethylamino)ethyl Acrylat, 2-(N,N-Dimethylamino)ethyl Methacrylate, 2-(N,N-Diethylamino)ethyl Acrylate, 2-(N,N-Diethylamino)ethyl Methacrylate, 3-(N,N-Dimethylamino)propyl-Acrylat, 3-(N,N-Dimethylamino)propyl-Methacrylat, 2-(N,N-Dimethylamino)neopentyl Acrylat, N'-(3-N,N-Dimethylamino)propyl-Acrylamid, N'-(3-N,N-Dimethylamino)propyl-Methacrylamid.

Bevorzugt sind Copolymere A, gebildet aus
- mindestens einem Monomer (A1) aus der Gruppe Acrylsäure, Methacrylsäure, C₁-C₆-Alkylacrylsäureester, C₁-C₆-Alkylmethacrylsäureester,
- mindestens einem Monomer (A2) aus der Gruppe der C₁₀₋₃₀Alkyl-PEG 20-Itaconate
- mindestens eine Monomer (A3) aus der Gruppe Acrylamid, Methacrylamid, Mono-(C₁-C₄)-alkylamino(C₁-C₄)-alkylacrylat, Di-(C₁-C₄)-alkylamino(C₁-C₄)-alkylacrylat, Mono-(C₁-C₄)-alkylamino(C₁-C₄)-alkylmethacrylat, Di-(C₁-C₄)-alkylamino(C₁-C₄)-alkylmethacrylat,

Besonders bevorzugt eingesetzte Copolymere a) sind quervernetzt. Durch die Quervernetzung werden die haptischen Eigenschaften der erfindungsgemäß eingesetzten Mittel verbessert. Zudem wird die haarfixierende Wirkung dieser Mittel, insbesondere bei Verwendung kleiner Mengen, verbessert.

Zusammenfassend sind erfindungsgemäß bevorzugt verwendete kosmetische Zusammensetzungen dadurch gekennzeichnet, dass das Copolymer a) ausgewählt ist aus der Gruppe der Verbindungen mit den INCI Bezeichnungen Acrylates/C₁₀₋₃₀Alkyl Acrylate Crosspolymer, Acrylates/Steareth-20 Methacrylate Crosspolymer, Acrylates/Steareth-20 Methacrylate Copolymer, Acrylates/Beheneth-25 Methacrylate Copolymer, Acrylates/Steareth-20 Itaconate Copolymer, Acrylates/Ceteth-20 Itaconate Copolymer, Acrylates/Palmeth-25 Acrylate Copolymer, Acrylates/Aminoacrylates/C₁₀₋₃₀Alkyl PEG-20 Itaconat Copolymer. Entsprechende Polymere sind beispielsweise unter den Handelsnamen Ultrez^{®} 21, Pemulen^{®} TR1,Aculyn^{®} 22, Aculyn^{®} 28, Aculyn^{®} 88, Structure^{®} 2001, Structure^{®} 3001, Synthalen^{®} W2000 und Structure^{®} Plus erhältlich. Mit besonderem Vorzug ist das Copolymer a) ausgewählt aus der Gruppe der Verbindungen mit der INCI Bezeichnung Acrylates/C10-30 Alkyl Acrylate Crosspolymer.

Als zweiten wesentlichen Bestandteil enthalten die erfindungsgemäß verwendeten kosmetischen Zusammensetzungen mindestens ein hydrophob modifiziertes Polysaccharid aus der Gruppe der Stärke Octenylsuccinate b). Der Gewichtsanteil des Polysaccharids b) am Gesamtgewicht der kosmetischen Zusammensetzung beträgt 0,1 bis 8,0 Gew.-%, besonders bevorzugt 0,5 bis 6,0 Gew.-% und insbesondere 1,0 bis 4,0 Gew.-%.

Besonders bevorzugt verwendete kosmetische Zusammensetzungen sind dadurch gekennzeichnet, dass das Polysaccharid b) ausgewählt ist der Gruppe der Stärke Octenylsuccinate, inbesondere aus den Verbindungen mit der INCI Bezeichnung Natrium Starch Octenylsuccinate, vorzugsweise jedoch aus der Gruppe mit der INCI Bezeichnung Aluminium Starch Octenylsuccinate.

Die Stärkeester haben sich den Stärkeethern als hinsichtlich ihrer kosmetischen und haptischen Eigenschaften überlegen erwiesen.

Für die erfindungsgemäße Verwendung hat es sich als vorteilhaft erwiesen, bestimmte Gewichstverhältnisse zwischen dem Copolymer a) und dem Polysaccharid b) einzuhalten. Es ist daher erfindungsgemäß bevorzugt, wenn das Gewichtsverhältnis des Copolymers a) zum Polysaccharid b) 2:1 bis 1:30, vorzugsweise 1:1 bis 1:20 und insbesondere 1:2 bis 1:10 beträgt.

Neben den zuvor beschriebenen Copolymeren a) und Polysacchariden b) können die erfindungsgemäß verwendeten kosmetischen Zusammensetzungen weitere Wirk-, Hilfs- und Pflegestoffe enthalten.

Zusammenfassend finden solche kosmetischen Zusammensetzung Verwendung, die, bezogen auf ihr Gesamtgewicht, 0,1 bis 10 Gew.-%, vorzugsweise 0,2 bis 8,0 Gew.-% und insbesondere 0,5 bis 5,0 Gew.-% mindestens eines Wachses aus der Gruppe Bienenwachs und Carnaubawachs enthalten. Durch den Zusatz von Wachs zu den erfindungsgemäß verwendeten kosmetischen Zubereitungen konnte eine unerwartete Volumenvergrößerung der temporär verformten keratinischen Fasern erreicht werden.

Ein weiterer bevorzugter Bestandteil erfindungsgemäß verwenderter kosmetischen Zusammensetzungen bildet die Gruppe der filmbildenden Polymere. Diese filmbildenden Polymere sind dabei nicht identisch mit dem zuvor beschriebenen hydrophob modifizierten (Meth)acrylsäure Copolymer a). Der Gewichtsanteil des filmbildenden Polymers am Gesamtgewicht der kosmetischen Zusammensetzung beträgt vorzugsweise 0,1 bis 8,0 Gew.-%, bevorzugt 0,5 bis 6,0 Gew.-% und inbesondere 1,0 bis 4,0 Gew.-%.

Als filmbildende Polymere werden mit besonderem Vorzug nichtionische Polymere eingesetzt. Geeignete nichtionische Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) vertrieben werden.
- Schellack.
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Erfindungsgemäß bevorzugt sind insbesondere die Polyvinylpyrrolidone (INCI-Bezeichnung: PVP).

Als Pflegestoff können Proteinhydrolysate und/oder deren Derivate eingesetzt werden. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Ein weitere Gruppe von Pflegestoffen sind die Vitamine, Provitamine, Vitaminvorstufen und/oder deren Derivate. Dabei sind erfindungsgemäß solche Vitamine, Provitamine und Vitaminvorstufen bevorzugt, die üblicherweise den Gruppen A, B, C, E, F und H zugeordnet werden.

Weitere Pflegestoffe sind Glycerin, Propylenglykol, Panthenol und Sorbitol.

Als Pflegestoff können Pflanzenextrakt, aber auch Mono- bzw. Oligosaccharide und/oder Lipide eingesetzt werden.

Die Ölkörper bilden eine weitere Gruppe von Pflegestoffen. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen. Bevorzugte werden weiterhin Ölkörper aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Esteröle, das heißt Ester von C₆ - C₃₀ - Fettsäuren mit C₂ - C₃₀ - Fettalkoholen, vorzugsweise Monoester der Fettsäuren mit Alkoholen mit 2 bis 24 C-Atomen wie beispielsweise Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinat/- caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

Der Wassergehalt mit Vorzug verwendeter kosmetischer Zusammensetzungen beträgt 30 bis 80 Gew.-%, bevorzugt 40 bis 70 Gew.-% und insbesondere 45 bis 65 Gew.-%.

Die Zusammensetzung einiger verwendeter kosmetischer Mittel kann den folgenden Tabellen entnommen werden (Angaben in Gew.-% bezogen auf das Gesamtgewicht des kosmetischen Mittels sofern nicht anders angegeben).

| | Formel 86 | Formel 87 | Formel 88 | Formel 89 | Formel 90 |
|---|---|---|---|---|---|
| Acrylates/C10-30 Alkyl Acrylate Crosspolymer | 0,05 bis 5,0 | 0,1 bis 4,0 | 0,2 bis 2,0 | 0,6 | 0,3 |
| Aluminium Starch Octenylsuccinate | 0,1 bis 8,0 | 0,5 bis 6,0 | 1,0 bis 4,0 | 2,7 | 1,2 |
| Bienenwachs | 0,1 bis 10 | 0,2 bis 8,0 | 0,5 bis 5,0 | 3,0 | 8,7 |
| Misc | add 100 | add 100 | add 100 | add 100 | add 100 |

| | Formel 136 | Formel 137 | Formel 138 | Formel 139 | Formel 140 |
|---|---|---|---|---|---|
| Acrylates/C10-30 Alkyl Acrylate Crosspolymer | 0,05 bis 5,0 | 0,1 bis 4,0 | 0,2 bis 2,0 | 0,6 | 0,3 |
| Aluminium Starch Octenylsuccinate | 0,1 bis 8,0 | 0,5 bis 6,0 | 1,0 bis 4,0 | 2,7 | 1,2 |
| Bienenwachs | 0,1 bis 10 | 0,2 bis 8,0 | 0,5 bis 5,0 | 3,0 | 8,7 |
| Wasser | 30 bis 80 | 40 bis 70 | 45 bis 65 | 62 | 33 |
| Misc | add 100 | add 100 | add 100 | add 100 | add 100 |

| | Formel 171 | Formel 172 | Formel 173 | Formel 174 | Formel 175 |
|---|---|---|---|---|---|
| Acrylates/C10-30 Alkyl Acrylate Crosspolymer | 0,05 bis 5,0 | 0,1 bis 4,0 | 0,2 bis 2,0 | 0,6 | 0,3 |
| Aluminium Starch Octenylsuccinate | 0,1 bis 8,0 | 0,5 bis 6,0 | 1,0 bis 4,0 | 2,7 | 1,2 |
| Bienenwachs | 0,1 bis 10 | 0,2 bis 8,0 | 0,5 bis 5,0 | 3,0 | 8,7 |
| filmbildendes Polymer * | 0,1 bis 8,0 | 0,5 bis 6,0 | 1,0 bis 4,0 | 1,2 | 3,8 |
| Wasser | 30 bis 80 | 40 bis 70 | 45 bis 65 | 62 | 33 |
| Misc | add 100 | add 100 | add 100 | add 100 | add 100 |

| | Formel 181 | Formel 182 | Formel 183 | Formel 184 | Formel 185 |
|---|---|---|---|---|---|
| Acrylates/C10-30 Alkyl Acrylate Crosspolymer | 0,05 bis 5,0 | 0,1 bis 4,0 | 0,2 bis 2,0 | 0,6 | 0,3 |
| Aluminium Starch Octenylsuccinate | 0,1 bis 8,0 | 0,5 bis 6,0 | 1,0 bis 4,0 | 2,7 | 1,2 |
| Bienenwachs | 0,1 bis 10 | 0,2 bis 8,0 | 0,5 bis 5,0 | 3,0 | 8,7 |
| Polyvinylpyrrolidon | 0,1 bis 8,0 | 0,5 bis 6,0 | 1,0 bis 4,0 | 1,2 | 3,8 |
| Wasser | 30 bis 80 | 40 bis 70 | 45 bis 65 | 62 | 33 |
| Misc | add 100 | add 100 | add 100 | add 100 | add 100 |

## Patentansprüche

1. Verwendung einer kosmetischen Zusammensetzung, enthaltend
a) 0,05 bis 5,0 Gew.-% mindestens eines hydrophob modifizierten (Meth)acrylsäure Copolymers, das aus
- mindestens einem Monomer (A1) aus der Gruppe Acrylsäure, Methacrylsäure, C₁-C₆-Alkylacrylsäureester, C₁-C₆-Alkylmethacrylsäureester,
- mindestens einem Monomer (A2) aus der Gruppe der der C₁₀-₃₀Alkyl Acrylate, Steareth-20 Methacrylate, Beheneth-25 Methacrylate, Steareth-20 Itaconate, Ceteth-20 Itaconate, Palmeth-25 Acrylate oder C₁₀-₃₀Alkyl PEG-20 Itaconate gebildet wird;
b) 0,1 bis 8,0 Gew.-% mindestens eines hydrophob modifizierten Polysaccharids aus der Gruppe der Stärke Octenylsuccinate;
c) 0,1 bis 10 Gew.-% mindestens eines Wachses aus der Gruppe Bienenwachs und Carnaubawachs
zur temporären Verformung keratinhaltiger Fasern, insbesondere menschlicher Haare.

2. Verwendung nach Anspruch 1, **dadurch gekennzeichnet**, das Copolymer a) quervernetzt ist.

3. Verwendung nach einem der vorherigen Ansprüche, **dadurch gekennzeichnet, dass** das Copolymer a) ausgewählt ist aus der Gruppe der Verbindungen mit der INCI Bezeichnung Acrylates/C10-30 Alkyl Acrylate Crosspolymer.

4. Verwendung nach einem der vorherigen Ansprüche, **dadurch gekennzeichnet, dass** der Gewichtsanteil des Copolymers a) am Gesamtgewicht der kosmetischen Zusammensetzung 0,1 bis 4,0 Gew.-% und insbesondere 0,2 bis 2,0 Gew.-% beträgt.

5. Verwendung nach einem der vorherigen Ansprüche, **dadurch gekennzeichnet, dass** das Polysaccharid b) ausgewählt ist aus der Gruppe der Stärkeester.

6. Verwendung nach einem der vorherigen Ansprüche, **dadurch gekennzeichnet, dass** das Polysaccharid b) ausgewählt ist aus den Verbindungen mit der INCI Bezeichnung Aluminium Starch Octenylsuccinate.

7. Verwendung nach einem der vorherigen Ansprüche, **dadurch gekennzeichnet, dass** der Gewichtsanteil des Polysaccharids b) am Gesamtgewicht der kosmetischen Zusammensetzung 0,5 bis 6,0 Gew.-% und insbesondere 1,0 bis 4,0 Gew.-% beträgt.

8. Verwendung nach einem der vorherigen Ansprüche, **dadurch gekennzeichnet, dass** die kosmetische Zusammensetzung, bezogen auf ihr Gesamtgewicht, 0,2 bis 8,0 Gew.-% und insbesondere 0,5 bis 5,0 Gew.-% mindestens eines Wachses aus der Gruppe Bienenwachs und Carnaubawachs enthält.

9. Verwendung nach einem der vorherigen Ansprüche, **dadurch gekennzeichnet, dass** die kosmetische Zusammensetzung, bezogen auf ihr Gesamtgewicht, 0,1 bis 8,0 Gew.-%, vorzugsweise 0,5 bis 6,0 Gew.-% und insbesondere 1,0 bis 4,0 Gew.-% mindestens eines von dem Copolymer a) verschiedenen filmbildenden Polymers, vorzugsweise mindestens eines filmbildenden Polymers aus der Gruppe der Polyvinylpyrrolidone enthält.

## Claims

1. The use of a cosmetic composition containing
a) from 0.05 to 5.0 wt.% of at least one hydrophobically modified (meth)acrylic acid copolymer that is formed from
- at least one monomer (A1) from the group comprising acrylic acid, methacrylic acid, C₁-C₆ alkyl acrylic acid esters, C₁-C₆ alkyl methacrylic acid esters,
- at least one monomer (A2) from the group comprising C₁₀-C₃₀ alkyl acrylates, steareth-20 methacrylate, beheneth-25 methacrylate, steareth-20 itaconate, ceteth-20 itaconate, palmeth-25 acrylate or C₁₀-₃₀ alkyl PEG-20 itaconates;
b) from 0.1 to 8.0 wt.% of at least one hydrophobically modified polysaccharide from the group comprising starch octenylsuccinates;
c) from 0.1 to 10 wt.% of at least one wax from the group comprising beeswax and carnauba wax,
for temporarily shaping keratin-containing fibers, in particular human hair.

2. The use according to claim 1, **characterized in that** copolymer a) is crosslinked.

3. The use according to one of the preceding claims, **characterized in that** the copolymer a) is selected from the group comprising the compounds having the INCI name Acrylates/C10-30 Alkyl Acrylate Crosspolymer.

4. The use according to one of the preceding claims, **characterized in that** the weight proportion of the copolymer a) in the total weight of the cosmetic composition is from 0.1 to 4.0 wt.% and in particular from 0.2 to 2.0 wt.%.

5. The use according to one of the preceding claims, **characterized in that** the polysaccharide b) is selected from the group comprising starch esters.

6. The use according to one of the preceding claims, **characterized in that** the polysaccharide b) is selected from the compounds having the INCI name Aluminum Starch Octenylsuccinate.

7. The use according to one of the preceding claims, **characterized in that** the weight proportion of the polysaccharide b) in the total weight of the cosmetic composition is from 0.5 to 6.0 wt.% and in particular from 1.0 to 4.0 wt.%.

8. The use according to one of the preceding claims, **characterized in that** the cosmetic composition, based on its total weight, contains from 0.2 to 8.0 wt.% and in particular from 0.5 to 5.0 wt.% of at least one wax from the group comprising beeswax and carnauba wax.

9. The use according to one of the preceding claims, **characterized in that** the cosmetic composition, based on its total weight, contains from 0.1 to 8.0 wt.%, preferably from 0.5 to 6.0 wt.%, and in particular from 1.0 to 4.0 wt.% of at least one film-forming copolymer, preferably at least one film-forming copolymer from the group comprising polyvinylpyrrolidones, that is different from the copolymer a).

## Revendications

1. Utilisation d'une composition cosmétique contenant
a) 0,05 à 5,0 % en poids d'au moins un copolymère d'acide (méth)acrylique à modification hydrophobe, qui est constitué
- d'au moins un monomère (A1) issu du groupe comprenant l'acide acrylique, l'acide métacrylique, les esters d'acide alkylacrylique en C₁-C₆, les esters d'acide alkylméthacrylique en C₁-C₆,
- d'au moins un monomère (A2) issu du groupe comprenant les acrylates d'alkyle en C₁₀₋₃₀, Stéareth-20 Méthacrylates, Béhéneth-25 Méthacrylates, Stéareth-20 Itaconates Céteth-20 Itaconates, Palmeth-25 Acrylates ou Alkyl PEG-20 Itaconates en C₁₀₋₃₀ ;
b) 0,1 à 8,0 % en poids d'au moins un polysaccharide modifié par hydrophobie du groupe de l'octénylsuccinate d'amidon ;
c) 0,1 à 10 % en poids d'au moins une cire issue du groupe comprenant la cire d'abeille et la cire de Carnauba
pour la mise en forme temporaire de fibres kératiniques, en particulier de cheveux humains.

2. Utilisation selon la revendication 1 **caractérisée en ce que** le copolymère a) est réticulé.

3. Utilisation selon l'une quelconque des revendications précédentes **caractérisée en ce que** le copolymère a) est sélectionné dans le groupe des composés portant le nom INCI Acrylates/C10-30 Alkyl Acrylate Crosspolymer.

4. Utilisation selon l'une quelconque des revendications précédentes **caractérisée en ce que** la part en poids du copolymère a) dans le poids total de la composition cosmétique représente 0,1 à 4,0 % en poids et en particulier 0,2 à 2,0 % en poids.

5. Utilisation selon l'une quelconque des revendications précédentes **caractérisée en ce que** le polysaccharide b) est sélectionné dans le groupe des esters d'amidon.

6. Utilisation selon l'une quelconque des revendications précédentes **caractérisée en ce que** le polysaccharide b) est sélectionné parmi les composés portant le nom INCI Aluminium Starch Octenylsuccinate.

7. Utilisation selon l'une quelconque des revendications précédentes **caractérisée en ce que** la part en poids du polysaccharide b) dans le poids total de la composition cosmétique représente 0,5 à 6,0 % en poids et en particulier 1,0 à 4,0 % en poids.

8. Utilisation selon l'une quelconque des revendications précédentes **caractérisée en ce que** la composition cosmétique contient, par rapport à son poids total, 0,2 à 8,0 % en poids et en particulier 0,5 à 5,0 % en poids d'au moins une cire issue du groupe comprenant la cire d'abeille et la cire de Carnauba.

9. Utilisation selon l'une quelconque des revendications précédentes **caractérisée en ce que** la composition cosmétique contient, par rapport à son poids total, 0,1 à 8,0 % en poids, de préférence 0,5 à 6,0 % en poids et en particulier 1,0 à 4,0 % en poids d'au moins un polymère filmogène différent du copolymère a), de préférence d'au moins un polymère filmogène du groupe de la polyvinylpyrrolidone.
